# EUROPÄISCHE PATENTANMELDUNG

(11) **EP 4 201 465 A1**
(43) Veröffentlichungstag der Anmeldung: **28.06.2023**
(21) Anmeldenummer: 22214062.6
(22) Anmeldetag: 16.12.2022
(51) Int. Cl.: A61M 27/00, A61M 25/00, A61M 25/02

(54) **HARNKATHETERVORRICHTUNG**

(30) Priorität: 22.12.2021 DE 102021214927
(71) Anmelder: Manfred Sauer GmbH, 74931 Lobbach (DE)
(72) Erfinder: Braner, Braner, 69251 Gaiberg (DE)
(74) Vertreter: Ullrich & Naumann PartG mbB

(57) **Zusammenfassung**

Eine Kathetervorrichtung (1) mit einem flexiblen Katheterschlauch (2), und einem äußeren Haltemittel (4) zum Fixieren von mindestens einem Teil des Katheterschlauchs (2) in der Harnröhre und der Blase im eingeführten Zustand. Ein Kit umfassend einen Katheter (2) und ein äußeres Haltemittel (4).

## Beschreibung

Die Erfindung betrifft eine Kathetervorrichtung mit einem flexiblen Katheterschlauch, und einem äußeren Haltemittel zum Fixieren von mindestens einem Teil des Katheterschlauchs in der Harnröhre und der Blase im eingeführten Zustand und ein Kit umfassend einen Katheter und ein äußeres Haltemittel.

Für die Entleerung der Blase bei Inkontinenz, bei etwa querschnittsgelähmten Menschen, werden üblicherweise Blasenkatheter verwendet. Sofern die Mobilität der Hände es zulässt, kann der Anwender den Blasenkatheter selbst anlegen. Bei nicht mobilen Menschen wird der Katheter von medizinischem Fachpersonal gelegt.

Ein modernes Verfahren zur Blasenentleerung ist der intermittierende Selbstkatheterismus. Hierbei wird mehrmals täglich ein Einmalkatheter in die Harnblase eingeführt, um diese zu entleeren. Ist der Urin vollständig abgelaufen, wird der Katheter wieder entfernt und entsorgt. Der Katheter verweilt also nur eine kurze Zeit im Körper des Anwenders. Abhängig von der Flüssigkeitsaufnahme, sollte die Blase vier bis sechsmal am Tag entleert werden. Das führt dazu, dass eine Blasenentleerung mittels intermittierenden Selbstkatheterismus auch nachts durchgeführt werden muss und der Anwender nicht durchschlafen kann. Obwohl viele Anwender versuchen, den gesamten Prozess der Blasenentleerung mittels intermittierenden Selbstkatheterismus in der Nacht so kurz wie möglich zu halten, haben die meisten von ihnen Probleme wieder einzuschlafen. Dauerhaft wirkt sich der reduzierte und wenig erholsame Schlaf negativ auf die körperliche und psychische Verfassung des Anwenders aus. Ein Belassen des Einmalkatheters in der Harnröhre bzw. der Blase über Nacht ist nicht möglich, da der Katheter durch die Blase selbst und die Schleimhaut der Harnröhre mit der Zeit aus ebendiesen heraustransportiert wird.

Menschen, die sich nicht selbst katheterisieren können oder die nicht mehrmals täglich, insbesondere nachts, einen Katheter zur Blasenentleerung verwenden wollen, können einen Dauerkatheter gelegt bekommen. Der Dauerkatheter wird über die Harnröhre eingeführt und ist mit einem inneren Haltemittel, üblicherweise einem Ballon, versehen, der den Katheter in der Blase fixiert und somit das Herausgleiten des Katheterschlauchs aus der Blase verhindert. Ein Dauerkatheter verbleibt üblicherweise über mehrere Wochen im Körper des Anwenders. Dadurch ergeben sich einige Risiken. Zwischen Harnröhre und Katheterschlauch bildet sich zwangsläufig mit längerer Verweildauer des Katheters im Körper ein Bakterienfilm. Die Bakterien können Infektionen hervorrufen, die die Harnröhre, die Blase und/oder die Nieren betreffen können. Um dem vorzubeugen, kann der Dauerkatheter regelmäßig mit einer desinfizierenden Lösung gespült werden. Eine regelmäßige Spülung der Blase ist außerdem nötig. Außerdem kann der Katheter bei längerer Liegedauer durch Verkrustung verstopfen und eine Harnableitung ist nicht mehr möglich.

Der vorliegenden Erfindung liegt daher die Aufgabe zugrunde, eine Kathetervorrichtung bereitzustellen, die über mehrere Stunden im Körper, insbesondere in der Blase, des Anwenders verbleiben kann und ein geringes Risiko für Komplikationen, insbesondere Infektionen und/oder Verkrustungen, aufweist.

Erfindungsgemäß wird die voranstehende Aufgabe durch die Merkmale des Anspruches 1 gelöst. Danach hat die in Rede stehende Kathetervorrichtung zur Harnableitung einen flexiblen Katheterschlauch und ein äußeres Haltemittel zum Fixieren von mindestens einem Teil des Katheterschlauchs in der Harnröhre und der Blase im eingeführten Zustand.

In erfindungsgemäßer Weise ist zunächst erkannt worden, dass auf eine separate Befestigung eines Katheterschlauchs mit einem inneren Haltemittel in der Blase verzichtet werden kann, um einen Katheterschlauch für einen mehrstündigen Zeitraum in der Blase zu fixieren. Die Fixierung des Katheterschlauchs im eingeführten Zustand ist über ein äußeres Haltemittel möglich, das ein Heraustransportieren des Katheterschlauchs verhindert.

Dadurch wird erreicht, dass der Anwender, insbesondere nachts, keine zusätzliche Selbstkatheterisierung vornehmen muss, um die Blase zu entleeren. Der Anwender kann den Katheterschlauch der erfindungsgemäßen Kathetervorrichtung vor dem Zubettgehen einführen und mit dem äußeren Haltemittel im Schambereich, wie etwa außen am Penis und/oder am Skrotum befestigen. Die Kathetervorrichtung kann auch mit dem äußeren Haltemittel an einem der Oberschenkel, mindestens jedoch in der räumlichen Nähe zum Penis, befestigt werden. Ein Durchschlafen in der Nacht ist somit gewährleistet.

Die erfindungsgemäße Kathetervorrichtung kommt ohne innere Haltemittel aus, die den Katheterschlauch innerhalb des Körpers des Anwenders, wie etwa in der Blase, befestigen, wie etwa Ballonstrukturen, die nach dem Einführen des Katheterschlauchs in die Blase aufgebaut werden. Somit ist auch ein im Vergleich zu Dauerkathetern kürzere Verweilzeit in der Harnröhre bzw. der Blase möglich. In einer Ausführungsform weist die Kathetervorrichtung demnach kein inneres Haltemittel zum fixieren des Katheterschlauchs in der Blase auf.

Die Kathetervorrichtung ist insbesondere vorteilhaft für eine Verweilzeit von mehreren Stunden, da keine Infektionen durch Bakterienbefall der Harnröhre, Blase und/oder Nieren zu befürchten sind. Die Verweilzeit von mehreren Stunden, etwa über die Nacht, ist für eine entsprechende Vermehrung der Bakterien und der Bildung eines Bakterienfilms zwischen Katheterschlauch und Harnröhre bzw. Blase zu kurz. Vorkehrungen, die über die üblichen Vorbereitungshandlungen zur Einführung eines Katheters, insbesondere eines intermittierenden Selbstkatheters, hinausgehen, sind folglich nicht erforderlich.

Nichtsdestotrotz kann es nötig sein, vor dem Einführen des Katheterschlauchs oder während der Katheterschlauch in der Harnröhre und/oder der Blase verweilt, eine Desinfektion durchzuführen. Dies kann beispielsweise durch desinfizierende Wirkstoffe, wie Antibiotika, antivirale Mittel oder ähnliches geschehen, die auf der Oberfläche des Katheterschlauchs aufgebracht sind oder durch den Katheter in die Blase geleitet werden können.

In einer Ausführungsform weist der Katheterschlauch eine am einführseitigen Ende des Katheterschlauchs fest angeordnete Einführhilfe zum Einführen in die Harnröhre bzw. zum Durchführen in die Blase auf.

Eine Einführhilfe erleichtert das Einführen des Katheterschlauchs in die Harnröhre und vermindert gleichzeitig die Wahrscheinlichkeit von Verletzungen bei diesem Vorgang, die durch zu große Kraftausübung auftreten können. Die Einführhilfe kann im Wesentlichen mit gleichem Durchmesser an den Schlauch anschließen und mit einem abgerundeten Kopfbereich enden. Der Kopfbereich kann dabei einen geringfügig größeren Durchmesser aufweisen als der Durchmesser des Schlauchs. Damit wird erreicht, dass beim Einführen der Katheterspitze in die Harnröhre diese aufgrund der Ausgestaltung des abgerundeten, kugeligen Kopfbereichs zumindest geringfügig gedehnt wird, so dass Taschen, Faltungen und Bögen wesentlich einfach zu überwinden sind.

Die Einführhilfe kann aus einem ähnlichen Material wie der Schlauch gefertigt sein, wobei die Einführhilfe insgesamt eine geringere Festigkeit als der Schlauch aufweist.

Ein äußeres Haltemittel dient für die Fixierung des Katheterschlauchs im eingeführten Zustand. Damit wird vermieden, dass der Katheter mit der Zeit aus der Blase heraustransportiert wird und eine Entleerung der Blase nicht mehr stattfinden kann. Das äußere Haltemittel wird für die Fixierung außen auf zumindest einen Teil des Penis, wie etwa der Eichel oder zumindest einem Abschnitt des Penisschafts, und/oder dem Skrotum befestigt. Das Haltemittel kann auch an einem Oberschenkel des Verwenders befestigt werden, mindestens jedoch in räumlicher Nähe zum Penis. Damit der Katheterschlauch fixiert werden kann, muss das äußere Haltemittel außerdem mit dem Katheterschlauch selbst oder zumindest einem Bestandteil der Kathetervorrichtung zusammenwirken. Eine entsprechende Verbindung zum Katheterschlauch und/oder der Kathetervorrichtung muss vorliegen.

Die Haut im Genitalbereich ist sehr empfindlich. Ein häufiger Gebrauch eines Haltemittels kann auch für die Haut am Oberschenkel irritierend/reizend sein. Daher muss das äußere Haltemittel schonend für die Haut sein. Kritisch ist hierbei, trotzdem eine ausreichende Befestigung des Katheterschlauchs bzw. der Kathetervorrichtung zu gewährleisten. In einer Ausführungsform wirkt das äußere Haltemittel auf Grundlage von Adhäsion, Formschluss, Stoffschluss, Haftreibung, Vakuum, Klettwirkung oder Kombinationen davon.

Die Wirkung aufgrund von Adhäsion bezieht sich auf die Anhaftung zweier Oberflächen durch einen oder mehrere Klebstoffe oder Haftstoffe, die zwischen den beiden Oberflächen aufgebracht sind. Die Adhäsion bezieht sich daher auf die Anhaftung durch Molekularkräfte. Ähnlich verhält es sich bei dem Stoffschluss, wobei zwei Oberflächen mittels eines Klebers miteinander verbunden werden.

Das Haltemittel kann ebenso über ein Vakuum wirken. Als Vakuum ist bereits ein geringer Unterdruck zu verstehen, der dazu führt, dass das Haltemittel beispielsweise am Penis festgehalten wird. Dazu verfügt das Haltemittel über einen Anschluss führ eine Vakuumpumpe oder wird kurz vor der Befestigung am Penis evakuiert.

Das Haltemittel kann außerdem über Haftreibung wirken. Das bedeutet, dass das Haltemittel eine Kraft auf den Körperteil, wie dem Penis oder den Oberschenkel auswirkt, sodass ein Verrutschen des Katheterschlauchs nicht möglich ist. Die Kraft, die dabei auf den Körperteil wirkt muss jedoch derart gewählt werden, dass die Durchblutung des Körperteils nicht gestört ist.

Das Haltemittel kann auch dermaßen ausgestaltet sein, dass es formschlüssig mit dem Oberschenkel, dem Penis oder eines Teils davon ist. Dadurch ergibt sich ein enganliegendes Konstrukt aus Haltemittel und Penis oder Oberschenkel, was ein Verrutschen des Haltemittels und damit des Katheterschlauchs ebenfalls ausschließt.

Das Haltemittel kann auch mittels Klettwirkung ein Verrutschen des Katheterschlauchs verhindern. Klettverbindungen sind einfach anzulegen und sind reversibel, was es für die mehrfache Verwendung qualifiziert.

Ebenfalls können Kombinationen der genannten Wirkungen vorteilhaft sein. Beispielsweis kann das Haltemittel eine selbstklebende Klettvorrichtung sein. Außerdem kann das Haltemittel ein Halteband mit Klettvorrichtung sein, wobei das Halteband mittels Formschluss oder Haftreibung an dem Körperteil befestigt ist.

Das Haltemittel kann zusätzliche Wirkstoffe enthalten, die vorzugsweise auf der dem Penis zugewandten Seite des Haltemittels vorliegen, wenn dieses am Penis angeordnet ist. Die Wirkstoffe haben keine primäre Haltefunktion. Das Haltemittel kann beispielsweise Medikamente und/oder Desinfektionsmittel enthalten. Möglich wären in etwa Medikamente, die Infektionen bekämpfen können, wie Antibiotika, antivirale Mittel oder ähnliches. Aufgrund der Feuchtigkeit im Genitalbereich können Pilzinfektionen auftreten. Das Haltemittel kann daher auch antimykotische Wirkstoffe enthalten. Außerdem kann die Haut oder die Harnröhre durch den häufigen Kontakt mit dem Katheterschlauch und/oder dem Haltemittel gereizt oder irritiert sein. Das Haltemittel kann beispielsweise pflegende Wirkstoffe enthalten. Ein Vorteil, dass das Haltemittel zusätzliche Wirkstoffe enthalten kann, ist der, dass damit lokal durch die Katheterisierung bzw. das Haltemittelauftretende Verletzungen/Infektionen der Haut direkt behandelt werden können, ohne die Katheterisierungsmethode zu ändern.

In einer weiteren Ausführungsform ist das äußere Haltemittel als eine elastische Hülle oder ein elastischer Schlauch ausgebildet, die/der über den Penis ziehbar und/oder abrollbar ist.

In vorteilhafter Weise ist die elastische Hülle oder der elastische Schlauch über den Penis ziehbar und/oder abrollbar. Durch die Elastizität wird gewährleistet, dass unabhängig von der Penisgröße eine Fixierung durch ein anliegendes Haltemittel gewährleistet ist. Durch die Fixierung des Katheterschlauchs mittels eines elastischen Schlauchs oder einer elastischen Hülle wird außerdem das Risiko von Infektionen weiter gesenkt. Die Hülle/der Schlauch umschließt den gesamten oberen Teil des Penis und verhindert somit, dass Bakterien entlang des Katheterschlauchs während der Anwendung in die Harnröhre gelangen.

In einer weiteren Ausführungsform ist das äußere Haltemittel als Kondom ausgestaltet. Abrollbare Kondome sind bekannt und können platzsparend und steril transportiert werden. Eine besondere Form des Kondoms, die für die Harnableitung bei Inkontinenz verwendet wird, ist das Urinalkondom. In einer weiteren Ausführungsform ist das äußere Haltemittel in der Form eines Urinalkondoms.

Ein Urinalkondom weist die Form eines Kondoms auf und verfügt über ableitende Anschlussmöglichkeiten, die vorzugsweise an der Spitze des Kondoms angeordnet sind. Als Anschlussmöglichkeit kommt insbesondere ein Schlauchansatz in Frage. Über diesen Schlauchansatz kann der Harn aus dem Urinalkondom in einen Schlauch und beispielsweise von dort aus in einen Urinbeutel transportiert werden. Ferner ist eine Verwendung mit einem Beinbeutel möglich, der sich an Oberschenkel oder Unterschenkel anbringen lässt. Gleiches gilt für einen Bettbeutel.

Die Verwendung eines Urinalkondoms bringt keine negativen Beeinträchtigungen wie etwa Hautirritationen, mangelnde Dichte oder unangenehme Gerüche mit sich.

Das Urinalkondom kann mittels Hautkleber, doppelseitigen Klebebändern oder als selbstklebende Variante am Penis befestigt werden.

Die Verwendung eines Hautklebers bietet den Vorteil, dass der Anwender die Menge, Breite und Hautstelle selbst bestimmen kann. Somit können bereits verletzte oder wunde Hautstellen ausgelassen werden. Hautkleber können in unterschiedlichen Klebestärken und Zusammensetzungen erworben werden. Sollten Unverträglichkeiten gegenüber bestimmten Bestandteilen von Hautklebern vorliegen, können daher alternative Kleberzusammensetzungen verwendet werden.

Hautkleber können pflegende Bestandteile aufweisen, wie etwa Lanolin, Zinkoxid, oder ähnliches. Des Weiteren können sich die Harzanteile im Kleber unterscheiden. Der Hautkleber kann beispielweise einen Harzanteil von 2 bis 15 Gew.-% aufweisen. Je höher der Harzanteil, desto höher ist die Klebekraft.

Leidet der Anwender unter einer Allergie gegen einen oder mehrere Bestandteile des eines Hautklebers, wird eine Alternative benötigt. Eine geeignete Alternative kann die Befestigung mit einem Klebeband, insbesondere einem doppelseitigen Klebeband sein. Auch hier können die Stelle der Klebung und die Größe der Klebefläche durch den Anwender bestimmt werden.

Die Befestigung des Urinalkondoms kann auch mittels selbstklebendem Urinalkondom geschehen. Das Urinalkondom kann dafür über eine selbsthaftende Schicht auf mindestens einem Abschnitt seiner Innenseite verfügen. Die Haftungseigenschaften gelten durch das gleichmäßige Auftragen als effizienter als bei anderen Fixierungen. Außerdem kann die Anwendung der selbstklebenden Exemplare leicht erlernt werden.

In einer weiteren Ausführungsform ist das äußere Haltemittel als Klebestreifen, Klettvorrichtung, Gummiband, Fixierbinde, Halteband oder Kombinationen davon ausgebildet.

Klebestreifen ermöglichen es, in der Länge variabel eingesetzt zu werden. Somit können sie je nach Anatomie des Anwenders, speziell auf diesen abgestimmt, verwendet werden. Klebestreifen ermöglichen somit eine Fixierung des Katheterschlauchs, die individuell auf den Anwender abgestimmt ist.

Die Klebestreifen können mit einem Klebstoff ausgestattet sein, der schonend zur Hautoberfläche ist. Außerdem sollte der Klebestreifen wasserdampfdurchlässig sein. Damit wird verhindert, dass sich Schweiß unter dem Klebestreifen sammelt und/oder der Klebestreifen durchweicht und sich von der Haut löst.

Je nach Anwender sind unterschiedliche Klebestärken der Klebestreifen gewünscht. Dies kann über unterschiedlich starke Kleber auf dem Klebestreifen gewährleistet werden. Außerdem können auch Klebestreifen mit unterschiedlichen Klebestärken in Kombination verwendet werden. Beispielsweise können Klebestreifen mit einer ersten, schwachen Klebestärke verwendet werden, um den Klebestreifen auf der Haut zu befestigen. Um den Klebestreifen mit schwacher Klebestärke an dem Katheterschlauch oder der Kathetervorrichtung zu befestigen kann zusätzlich ein oder mehrere Klebestreifen mit einer zweiten, stärkeren Klebestärke benutzt werden.

In einer weiteren Ausführungsform ist das äußere Haltemittel in der Form mindestens eines Pflasters, vorzugsweise Heftpflaster. Pflaster, bzw. Heftpflaster sind für den medizinischen Gebrauch ausgelegt und können somit in vorteilhafter Weise als äußeres Haltemittel an der Kathetervorrichtung verwendet werden.

Die äußere Haltevorrichtung kann auch in Form einer Klettvorrichtung sein. Dafür kann beispielsweise ein Klettband verwendet werden. Ein Klettband besteht üblicherweise aus zwei Teilen, nämlich einem Klettteil und einem Teil aus Flauschband. Ein Teil des Klettbands wird an einem Körperteil, wie etwa dem Oberschenkel oder dem Penisschaft, befestigt und der andere Teil an dem Katheterschlauch oder einem Teil der Kathetervorrichtung angebracht. Durch das Zusammenfügen beider Teile des Klettbands, ist der Katheterschlauch an dem entsprechenden Körperteil befestigt.

Außerdem kann das äußere Haltemittel ein Gummiband oder ähnliches sein. Ein Gummiband kann über den Katheterschlauch oder einen Bestandteil der Kathetervorrichtung und dem entsprechenden Körperteil, wie dem Penis oder dem Oberschenkel, gezogen werden. Aufgrund der Elastizität des Gummis sind unterschiedliche Stärken möglich, die einen unterschiedlich festen Sitz des Haltemittels ermöglichen.

Das äußere Haltemittel kann auch eine Fixierbinde sein, die über einen Teil des nichteingeführten Abschnitts des Katheterschlauchs oder eines Bestandteiles der Kathetervorrichtung gelegt wird und den Katheterschlauch somit am entsprechenden Körperteil festlegt und in der Harnröhre und der Blase fixiert.

Ebenfalls kommt ein Halteband als äußeres Haltemittel in Betracht, das über den entsprechenden Körperteil, wie den Penis oder einen Oberschenkel, gezogen, gelegt und/oder festgezurrt wird und an dem der Katheterschlauch mittels Klettverbindung, Haken, Druckknopf oder ähnlichem befestigt wird.

In einer weiteren Ausführungsform weist der Katheterschlauch einen Adapter mit einer Aufnahme für das nicht-einführseitige Ende des Katheterschlauchs auf, wobei der Adapter an diesem Ende fest angeordnet ist.

Der Adapter ermöglicht es unter anderem, den Katheterschlauch, der einen definierten Außendurchmesser aufweist, der bedingt durch die Anatomie der Harnröhre begrenzt ist, zur Ableitung des Urins in beispielsweise einen Urinbeutel oder ein Schlauchsystem zur Ableitung in ein WC anzuschließen. Dabei weisen die Verbindungsschläuche zu einem Urinbeutel bzw. die Schlauchsysteme zur Ableitung üblicherweise einen größeren Außendurchmesser auf, als der Katheterschlauch. Über den Adapter wird daher gewährleistet, eine auslaufsichere Verbindung zwischen dem Katheterschlauch und dem ableitenden Schlauch/Schlauchsystem herzustellen.

Der Adapter kann folglich mindestens zwei Schlauchenden aufnehmen. Dafür weist der Adapter auf der im eingeführten Zustand dem Penis zugewandten Seite, im Folgendem als die dem Penis zugewandte Seite bezeichnet, eine Aufnahme für das nicht-einführseitige Ende des Katheterschlauchs auf. Auf der dem Penis entsprechend abgewandten Seite kann der Adapter eine weitere Aufnahme für einen Schlauch oder ein Schlauchsystem zur Ableitung des Urins aufweisen. Diese Aufnahme kann derart ausgestaltet sein, dass der Schlauch/das Schlauchsystem in den Adapter aufgenommen werden kann oder dass der Schlauch/ das Schlauchsystem auf den Adapter aufgesteckt wird. Der Adapter kann kraft- und/oder formschlüssig mit dem Schlauch/Schlauchsystem verbunden sein. Zusätzlich oder alternativ kann der Adapter lösbar oder unlösbar mit dem Schlauch/Schlauchsystem verbunden sein.

Der Adapter kann eine Gesamtlänge im Bereich von 1 bis 4 cm, vorzugsweise im Bereich von 1,5 bis 3,5 cm aufweisen. Es hat sich gezeigt, dass die Handhabung bei Adaptern, die kleiner als 1 cm sind, für Anwender mit eingeschränkter Handmotorik zu schwer ist. Mit zunehmender Gesamtlänge und Größe des Adapters nimmt auch das Gewicht der Kathetervorrichtung insgesamt zu, was den Tragekomfort reduziert.

In einer weiteren Ausführungsform weist der Adapter einen vorgegebenen Bereich bzw. Abschnitt zur Verbindung mit einem äußeren Haltemittel auf. Dadurch kann eine genaue Kraftverteilung und verbesserte Fixierung des Katheterschlauchs in der Harnröhre und der Blase erfolgen. Ebenfalls kann somit ein fehlerhaftes Anlegen des äußeren Haltemittels und/oder Verrutschen des Haltemittels vermieden werden, was zu einem Herausrutschen des Katheterschlauchs aus der Blase und/oder der Harnröhre führen kann.

Der Bereich bzw. Abschnitt zur Verbindung mit einem äußeren Haltemittel kann dabei durch ringförmige Erhöhungen, Erhebungen, Verdickungen und/oder Wülste auf der Oberfläche des Adapters vorgegeben sein. Die ringförmigen Erhöhungen, Erhebungen, Verdickungen und/oder Wülste sind Bereiche des Adapters, die sich im Vergleich zum weiteren Körper des Adapters weiter nach außen erstrecken. Diese ringförmigen Erhöhungen, Erhebungen, Verdickungen und/oder Wülste können als Verbindungsbereich für das äußere Haltemittel dienen, indem sie beispielsweise mit dem Haltemittel formschlüssig zusammenwirken. Außerdem kann ein zwischen zwei oder mehreren ringförmigen Erhöhungen, Erhebungen, Verdickungen und/oder Wülsten liegender Bereich zur Verbindung mit dem äußeren Haltemittel dienen, wobei die ringförmigen Erhöhungen, Erhebungen, Verdickungen und/oder Wülste ein Verrutschen des Haltemittels verhindern.

In einer Ausführungsform ist die Aufnahme des Adapters für Katheterschläuche im Bereich von 10 bis 18 Charrière ausgelegt. In einer weiteren Ausführungsform ist der die Aufnahme des Adapters für Katheterschläuche mit 10, 12, 14, 16 oder 18 Charrière ausgelegt.

In einer weiteren Ausführungsform ist der Adapter mittels Spritzgusstechnik oder 3D Druck hergestellt worden. Somit lassen sich die Herstellungskosten für die Kathetervorrichtung senken.

Der Katheterschlauch kann mit dem Adapter kraft- oder formschlüssig zusammenwirken. Zusätzlich oder alternativ kann der Katheterschlauch mit dem Adapter verklebt sein. Als Kleber kommen medizinische Kleber mit einer hohen Klebekraft in Betracht. Des Weiteren ist es möglich, dass der Katheterschlauch mit dem Adapter verschweiß ist. Dafür kommt insbesondere die Verschweißung durch Ultraschallschweißung in Betracht. Ultraschallschweißen garantiert eine dreidimensionale Verschweißung des Katheterschlauchs mit dem Adapter. Undichtigkeiten sind dadurch praktisch ausgeschlossen. Außerdem kann auf den Einsatz von Klebern verzichtet werden, die möglicherweise zu Reizungen der Haut führen können. Ultraschallschweißen ist außerdem kostengünstig und lässt sich leicht in Produktionsstraßen integrieren.

In einer Ausführungsform ist der Katheterschlauch kraft- oder formschlüssig mit dem Adapter verbunden. In einer weiteren Ausführungsform ist der Katheterschlauch mit dem Adapter verklebt oder verschweißt.

In einer weiteren Ausführungsform weist der Adapter auf der dem Penis zugewandten Seite eine Stützstruktur zur Abstützung von zumindest einem Teil des Penis auf.

Die Stützstruktur verhindert, dass der Katheterschlauch oder weitere Teile der Kathetervorrichtung weiter als nötig in den Harnröhreneingang verrutscht. Dies ist insbesondere bei einer Erektion des Penis nötig, während der Katheterschlauch eingeführt und fixiert ist. Durch das Anschwellen des Penis und der entsprechenden Grö-ßen- und Volumenzunahme würde der Katheterschlauch unweigerlich in den Harnröhreneingang verrutschen und diesen aufspalten. Übliche Katheter weisen an dem nicht eingeführten Ende des Katheterschlauchs ein sich zum Schlauch verjüngenden, konisch geformten Konnektor zum Anschluss von Schlauchsystemen für Urinbeutel auf. Diese Art von Konnektoren würde bei einer Erektion in den Harnröhreneingang rutschen und diesen verletzen.

Die Stützstruktur des Adapters kann dabei auf der Penisspitze, insbesondere der Eichel, aufliegen oder dieser vorgelagert sein und verhindert das Hereinrutschen beim Anschwellen des Penis. Verletzungen des Harnröhreneingangs können somit vermieden werden.

Die Stützstruktur kann an die Anatomie des Penis, insbesondere der Penisspitze und/oder der Eichel, angepasst sein. Dadurch werden Druckstellen verhindert. Ausgehend von der Aufnahme für den Katheterschlauch verbreitert die Stützstruktur durchgängig oder unterbrochen den Rand der Aufnahme des Adapters, vorzugsweise relativ zum Durchmesser der Aufnahme bzw. des Katheterschlauchs. Entsprechend muss die Stützstruktur einen Durchmesser aufweisen, der größer ist als die Öffnung der Harnröhre, damit ein Hineinrutschen in diese vermieden wird.

In einer Ausführungsform weist die Stützstruktur einen Durchmesser im Bereich von 1 bis 2 cm, vorzugsweise im Bereich von 1 bis 1,5 cm, auf. Damit wird gewährleistet, dass eine Abstützung des Penis, insbesondere der Penisspitze, ermöglicht ist. Außerdem wird dem Tragekomfort weiter Rechnung getragen, wobei größere Durchmesser überstehen würden und vom Anwender als störend empfunden werden oder zu Hautirritationen durch Reibwirkung und Druckstellen an nebenliegenden Hautpartien entstehen können.

Die Stützstruktur kann in Form einer flachen Scheibe oder Platte vorliegen. Die Stützstruktur kann auch tellerförmig ausgestaltet sein. Die Stützstruktur kann auch umgekehrt Trichterförmig, sich also zur Penis abgewandten Seite verjüngend, kugelförmig, dreieckig oder kreuzförmig ausgestaltet sein. Des Weiteren kann die Stützstruktur individuell an die Form der Penisspitze, insbesondere der Eichelspitze, angepasst sein.

Die Stützstruktur in Form einer Platte oder Scheibe kann eine Höhe im Bereich von 0,1 bis 0,5 cm, vorzugsweise 0,2 bis 0,4 cm aufweisen. Damit wird gewährleistet, dass die Stützstruktur ausreichend stabil gegenüber Zug- und Haltekräften ist. Außerdem wird vermieden, dass mit geringerer Höhe eine Schneidwirkung des Rands der Stützstruktur auf den Penis ausgeübt wird, sodass Verletzungen vermieden werden.

In einer weiteren Ausführungsform ist die Aufnahme des nicht-einführseitigen Endes des Katheterschlauchs im Wesentlichen mittig durch die Stützstruktur geführt. Die Stützstruktur kann durchbrochen sein. Damit wird erreicht, dass die abstützende Funktion der Stützstruktur gleichmäßig auf die Penisspitze, insbesondere die Eichel wirkt. Dadurch werden Druckstellen durch unausgeglichenes Abstützen vorgebeugt. Außerdem ist eine Zentrierung des Adapters am oder vor dem Penis gewährleistet, was eine verbesserte Fixierung durch das Haltemittel ermöglicht.

In einer weiteren Ausführungsform ist das äußere Haltemittel zumindest teilweise mit dem Adapter lösbar oder unlösbar verbunden.

Zu Fixierung des Katheterschlauchs im eingeführten Zustand muss das äußere Haltemittel sowohl am Penis oder an einem penisnahen Körperteil, wie etwa einem Oberschenkel, des Anwenders als auch an der Kathetervorrichtung anliegen. Das äußere Haltemittel kann zumindest teilweise am Adapter anliegen. Es wird damit ermöglicht, dass das Haltemittel nicht direkt am Katheterschlauch befestigt sein muss. Bei gelbeschichteten oder bereits aktivierten hydrophilen Katheterschläuchen wäre es nötig, den nicht eingeführten Teil des Schlauchs von der Beschichtung zu befreien, bevor das äußere Haltemittel angebracht wird, um eine vollständige Befestigung zu gewährleisten. Außerdem kann der gesamte Katheterschlauch bis zum Adapter in die Harnröhre eingeführt werden.

Sofern das äußere Haltemittel Klebestreifen sind, kann der Adapter zur Befestigung der Klebestreifen geeignete einheitliche durchgängige Oberflächenabschnitte aufweisen. Damit wird gewährleistet, dass die Klebestreifen am Adapter ausreichend festkleben.

Bei einer elastischen Hülle bzw. einem elastischen Schlauch als äußerem Haltemittel kann der Adapter auf der Hülle oder dem Schlauch aufsitzen, sodass der Adapter bei abgerollter oder aufgezogener Hülle/Schlauch auf der Penisspitze aufsitzt. Die Hülle/ der Schlauch kann mit der dem Penis zugewandten Seite des Adapters verbunden sein. Insbesondere kann der Adapter mit der Hülle/ dem Schlauch verklebt oder verschweißt sein. Es ist außerdem denkbar, dass der Adapter direkt auf der Penisspitze aufsetzt und die Hülle/der Schlauch im ausgerollten Zustand über den Adapter gerollt/gezogen werden. Der Adapter kann somit zwischen Hülle/Schlauch und Penis sitzen.

Wenn das Haltemittel in Form eines Urinalkondoms vorliegt, kann das Urinalkondom über den Adapter gezogen sein oder der Adapter in das Urinalkondom gesteckt sein. Dabei ist der Adapter im Urinal zwischen dem ausrollbaren Abschnitt des Urinalkondoms und dem Schlauchansatz angeordnet. Der Adapter liegt dabei im eingeführten Zustand des Katheterschlauchs mit einer Seite, vorzugsweise der Stützstruktur, direkt auf der Penisspitze auf. Der Adapter kann dabei über seine gesamte Oberfläche oder einem Teil davon mit dem Urinalkondom verbunden sein. Dafür kann der Adapter mit dem Urinalkondom verschweißt oder verklebt sein. Außerdem ist es denkbar, dass der Adapter und das Urinalkondom kraft- und/oder formschlüssig miteinander verbunden sind.

In einer Ausführungsform ist das äußere Haltemittel ein Halteband mit einer Haltevorrichtung, wobei das Halteband an einem Oberschenkel des Anwenders befestigt ist. Das Halteband kann mit einem Teil einer Klettvorrichtung, Druckknöpfen oder ähnlichem ausgestattet sein. Zur Befestigung des Katheterschlauchs an dem Halteband, kann an dem Katheterschlauch oder an einem vordefinierten Bereich des Adapters die Haltevorrichtung angebracht sein. Die Haltevorrichtung kann ein zweiter Teil einer Klettvorrichtung, eine Schlaufe oder Lasche aus Stoff, Kunstfasern oder ähnlichem sein, der/die an dem Adapter festgelegt wird und an dem Halteband am Oberschenkel befestigt wird. Die Befestigung kann über Klettwirkung der beiden Teile der Klettvorrichtung oder Druckknöpfe oder ähnliches bewirkt werden.

In einer bevorzugten Ausführungsform ist das äußere Haltemittel die Kombination eines Haltebands mit einer Oberfläche, die ein Teil einer Klettvorrichtung ist, und einer Haltevorrichtung, die als Schlaufe über einen vorgegebenen Bereich eines Adapters am Katheterschlauch gelegt und festgezogen ist, wobei die Haltevorrichtung der zweite Teil einer Klettvorrichtung ist, der mit der Oberfläche des Haltebands zum Fixieren des Katheterschlauchs im eingeführten Zustand zusammenwirkt.

In einer weiteren Ausführungsform weist der Adapter auf der dem Penis abgewandten Seite einen Auslass zur Harnableitung und/oder Konnektierung mit einem Urinbeutel auf. Damit kann ein direktes Ableiten des Urins, beispielsweise in eine Toilette erfolgen. Der Adapter kann jedoch auch an einen Schlauch oder ein Schlauchsystem zum Ableiten des Urins in einen Urinbeutel angeschlossen sein. Für die Urinableitung in der Nacht ist der Adapter vorzugsweise mit einem Schlauch oder Schlauchsystem zur Ableitung in einen Urinbeutel verbunden.

Der Durchmesser des Auslasses des Adapters auf der dem Penis abgewandten Seite kann im Bereich zwischen 0,5 und 1,5 cm, vorzugsweise im Bereich von 0,9 bis 1,2 cm, liegen. Der Adapter kann sich von dem Auslass zur dem Penis gewandten Seite verjüngen und somit eine Konnektierung mit handelsüblichen Urinbeuteln ermöglichen.

Zum leichteren Einführen des Katheterschlauchs in die Harnröhre kann der Reibekoeffizient der Oberfläche des Schlauchs herabgesetzt werden. Das kann über das Beschichten der Katheteroberfläche mit einem Gel oder Schmiermittel geschehen. Alternativ kann die Oberfläche des Schlauchs mit einem hydrophilen Stoff versehen sein, der mit einem wasserhaltigen Medium aktiviert werden kann oder aktiviert worden ist. Somit wird erreicht, dass der Katheterschlauch ausreichend gleitfähig ist und das Einführen erleichtert wird.

In einer weiteren Ausführungsform weist der Katheterschlauch demnach eine gelbschichtete oder hydrophile Oberfläche auf.

In einer weiteren Ausführungsform hat der Katheterschlauch eine Länge im Bereich von 10 bis 40 cm, vorzugsweise im Bereich von 20 bis 40 cm. In einer weitere Ausführungsform hat der Katheterschlauch einen Außendurchmesser im Bereich von 2 bis 10 mm, vorzugsweise im Bereich von 3 bis 6 mm.

Im Gegensatz zu Dauerkathetern, die üblicherweise 2- oder 3-Wege-Katheter sind, ist der Katheterschlauch vorzugsweise als 1-Weg-Katheter, also mit nur einem Kanal, ausgestaltet. Dieser eine Kanal erlaubt die Harnableitung. 2-Wege-Katheter haben einen zusätzlichen Kanal zur Befüllung/Aktivierung des inneren Haltemittels, also der Ballonstruktur oder Ähnlichem. 3-Wege-Katheter haben einen weiteren Kanal zur Blasenspülung.

Die Erfindung betrifft außerdem einen Katheterschlauch für die hier beschriebene Kathetervorrichtung, wobei der Katheterschlauch einen Adapter mit einer Aufnahme für das nicht-einführseitige Ende des Katheterschlauchs aufweist, wobei der Adapter an diesem Ende fest angeordnet ist.

In einer Ausführungsform des Katheterschlauchs weist der Adapter auf der dem Penis zugewandten Seite eine Stützstruktur zur Abstützung von zumindest einem Teil des Penis auf.

In einer weiteren Ausführungsform weist der Adapter auf der dem Penis abgewandten Seite einen Auslass zur Harnableitung und/oder Konnektierung mit einem Urinbeutel auf.

Die Erfindung betrifft außerdem ein Kit, umfassend
- einen Katheter mit einem flexiblen Katheterschlauch, und
- ein äußeres Haltemittel zum Fixieren von mindestens einem Teil des Katheterschlauchs in der Harnröhre und der Blase im eingeführten Zustand.

Mit einem Kit können die Bestandteile zur Herstellung einer Kathetervorrichtung praktisch verpackt und transportiert werden. Das erfindungsgemäße Kit bietet außerdem die Möglichkeit sämtliche für die Katheterisierung nötigen Materialen räumlich zusammenzufassen. Dem Anwender können damit alle nötigen Bestandteile für die Katheterisierung bereitgestellt werden. Ein Zusammensuchen der Materialen entfällt für den Anwender.

Vorteilhaft ist auch, dass nicht sämtliche Materialen vor der Anwendung steril verpackt sein müssen. Während der Katheter, insbesondere der Katheterschlauch, steril sein muss, können weitere Bestandteile des Kits, wie etwa das äußere Haltmittel unsteril, sein. Die Kosten und der Aufwand bei der Herstellung eines entsprechenden Kits können somit geringgehalten werden.

In einer Ausführungsform des Kits weist der Katheterschlauch eine am einführseitigen Ende des Katheterschlauchs fest angeordnete Einführhilfe zum Einführen in die Harnröhre bzw. zum Durchführen in die Blase auf.

In einer weiteren Ausführungsform des Kits wirkt das äußere Haltemittel auf Grundlage von Adhäsion, Formschluss, Haftreibung, Vakuum oder Kombinationen davon.

In einer weiteren Ausführungsform des Kits ist das äußere Haltemittel als eine elastische Hülle bzw. ein elastischer Schlauch ausgebildet, die/der über den Penis ziehbar und/oder abrollbar ist.

In einer weiteren Ausführungsform des Kits ist das äußere Haltemittel als Klebestreifen, Klettvorrichtung, Gummiband, Fixierbinde, Halteband oder Kombinationen davon ausgebildet.

In einer weiteren Ausführungsform des Kits weist der Katheterschlauch einen Adapter mit einer Aufnahme für das nicht-einführseitigen Ende des Katheterschlauchs auf, wobei der Adapter an diesem Ende fest angeordnet ist.

In einer weiteren Ausführungsform des Kits weist der Adapter auf der dem Penis zugewandten Seite eine Stützstruktur zur Abstützung des Penis auf.

In einer weiteren Ausführungsform kann das Kit einen Urinauffangbeutel umfassen. Damit wird einem Anwender, der nicht oder nicht rechtzeitig ein WC aufsuchen kann, ermöglicht, den Urin in einen solchen Urinbeutel abzuleiten.

Das Kit kann ferner die einzelnen Bestandteile in einer gasdurchlässigen Verpackung enthalten. Die einzelnen Bestandteile können des Weiteren einzeln oder als funktionelle Einheiten in gasdurchlässigen Verpackungen angeordnet sein. Eine Sterilisation der Bestandteile ist dann durch die Begasung mit Ethylenoxid möglich. Die sterilisierten und verpackten Bestandteile können sodann in gasundurchlässige Umverpackungen verpackt werden. Dadurch wird ein austrocknen der Bestandteile verhindert.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: eine schematische Darstellung einer Ausführungsform der Kathetervorrichtung im Querschnitt.
- Fig. 2: eine schematische Darstellung einer Ausführungsform der Kathetervorrichtung im nicht-eingeführten (Fig. 2a) und eingeführten (Fig. 2b) Zustand im Querschnitt.
- Fig. 3: eine schematische Darstellung einer Ausführungsform des Adapters im Querschnitt.
- Fig. 4: eine schematische Darstellung einer Ausführungsform des Adapters im Querschnitt.
- Fig. 5: eine schematische Darstellung einer Ausführungsform der eingeführten Kathetervorrichtung im Querschnitt.

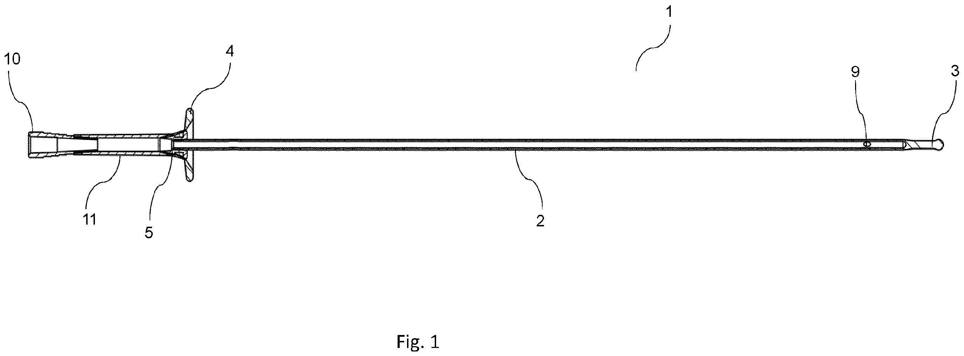

Fig. 1 zeigt eine schematische Darstellung einer Ausführungsform der Kathetervorrichtung 1 im Querschnitt mit einem flexiblen Katheterschlauch 2, einer Einführhilfe 3, die am einführseitigem Ende des Katheterschlauchs 2 angeordnet ist, und einem äußeren Haltemittel 4. Der Katheterschlauch 2 hat außerdem ein oder mehrere Ablaufaugen 9, die im Schlauchabschnitt vor der Einführhilfe 3 angeordnet sind. Das äußere Haltemittel 4 ist als Kondom, insbesondere ein Urinalkondom, ausgestaltet. Das Urinalkondom liegt im aufgerollten Zustand vor. Die Kathetervorrichtung 1 umfasst außerdem einen Schlauchansatz 11, der Teil des Urinalkondoms sein kann, oder separat am Adapter 5 festgelegt sein kann. Der Adapter 5 hat eine Aufnahme 6 für das nicht-eingeführte Ende des Katheterschlauchs 2 und einen Auslass 8 zur Ableitung des Urins. Der Schlauchansatz 11 kann mit einem Konnektor 10 zur Ableitung des Urins in einen Urinbeutel verbunden sein.

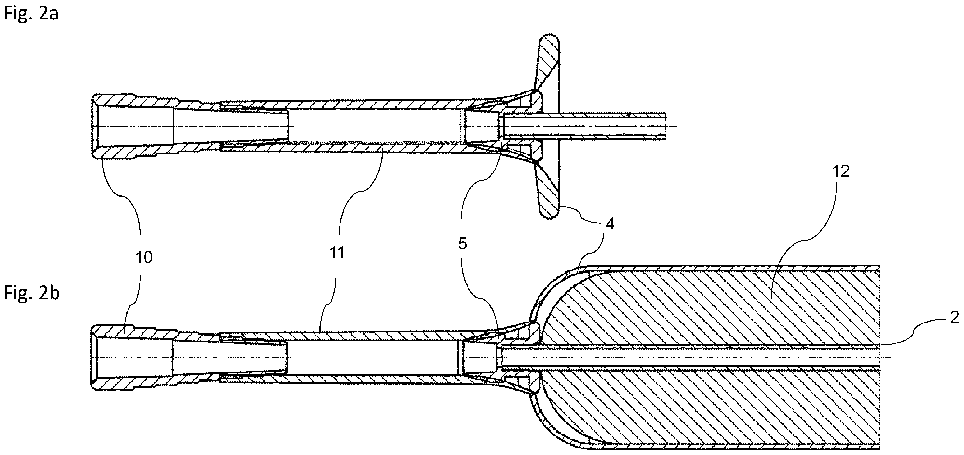

Fig. 2a zeigt eine schematische Darstellung des im eingeführten Zustand außenliegenden Teils der Kathetervorrichtung nach Fig. 1 im nicht-eingeführten Zustand im Querschnitt. Das äußere Haltemittel 4 ist zusammengerollt an der Kathetervorrichtung 1 angelegt. Das äußere Haltemittel 4 kann dabei mit den Adapter 5 kraft- und/oder formschlüssig verbunden sein. In Fig. 2b ist die Kathetervorrichtung 1 nach Fig. 2a im eingeführten Zustand abgebildet. Der Katheterschlauch 2 ist in die Harnröhre des Penis 12 eingeführt. Zur Fixierung des Katheterschlauchs 2 ist das äußere Haltemittel 4 abgerollt bzw. über einen Teil des Penis 12 gezogen worden und liegt an diesem an. Die Stützstruktur 7, liegt an der Penisspitze an.

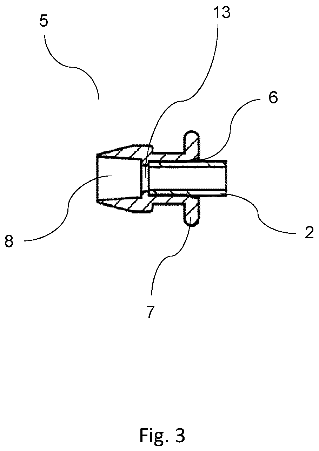

Fig. 3 zeigt eine schematische Darstellung einer Ausführungsform des Adapters 5 im Querschnitt. Der Adapter 5 hat auf der dem Penis zugewandten Seite eine Aufnahme 6 für das nicht-eingeführte Ende des Katheterschlauchs 2 und ist mit diesem fest verbunden. Der Adapter 5 weist auf der dem Penis zugewandten Seite außerdem eine Stützstruktur 7 auf, die im eingeführten Zustand auf dem Penis aufliegt und diesen abstützt. Dadurch wird ein Hineinrutsche der Kathetervorrichtung 1 in den Harnröhreneingang, beispielsweise bei einer Erektion, verhindert. Die Stützstruktur 7 ist im Wesentlichen flach ausgebildet und weist einen Durchbruch für die Aufnahme 6 auf, die vorzugsweise mittig durch die Stützstruktur 7 läuft. An der dem Penis abgewandten Seite weist der Adapter 5 einen Auslass 8 zum Ableiten des Urins in eine Toilette oder einen Schlauchansatz bzw. einen Urinbeutel auf. Der Urinbeutel kann über einen Schlauch oder ein Schlauchsystem mit dem Schlauchansatz oder dem Adapter 5 verbunden sein. Der Urinbeutel kann außerdem über einen Konnektor 10 mit dem Schlauchansatz 11 verbunden sein. Damit der Urin aus dem Katheterschlauch 2 zum Auslass 8 gelangt, ist im Adapter 5 eine Durchflussöffnung 13 vorgesehen.

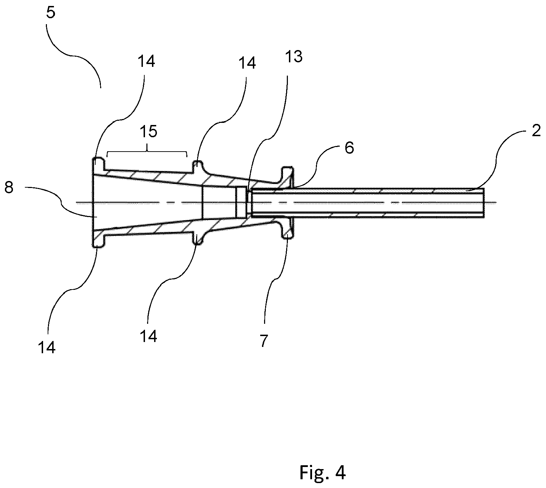

Fig. 4 zeigt eine schematische Darstellung einer Ausführungsform des Adapters 5 im Querschnitt. Der Adapter 5 hat auf der dem Penis zugewandten Seite eine Aufnahme 6 für das nicht-eingeführte Ende des Katheterschlauchs 2 und ist mit diesem fest verbunden. Der Adapter 5 weist auf der dem Penis zugewandten Seite außerdem eine Stützstruktur 7 auf, die im eingeführten Zustand auf dem Penis 12 aufliegt und diesen abstützt oder dem Penis 12 vorgelagert ist und im Falle einer Erektion auf der Penisspitze anliegt. Dadurch wird ein Hineinrutsche der Kathetervorrichtung 1 in den Harnröhreneingang verhindert. Die Stützstruktur 7 ist im Wesentlichen flach ausgebildet und weist einen Durchbruch für die Aufnahme 6 auf, die vorzugsweise mittig durch die Stützstruktur 7 läuft. An der dem Penis abgewandten Seite weist der Adapter 5 einen Auslass 8 zum Ableiten des Urins in eine Toilette oder einer Konnektierung mit einem Urinbeutel auf. Der Urinbeutel kann über einen Konnektor 10 mit dem Auslass 8 des Adapters 5 verbunden sein. Damit der Urin aus dem Katheterschlauch 2 zum Auslass 8 gelangt, ist im Adapter 5 eine Durchflussöffnung 13 vorgesehen.

In Fig. 4 weist der Adapter 5 ferner einen Abschnitt 15 zur Verbindung mit einem äußeren Haltemittel 4 auf. Der Abschnitt 15 ist durch zwei ringförmige Erhöhungen 14 auf der Oberfläche des Adapters 5 vorgegeben. Die ringförmigen Erhöhungen 14 verhindern ein Verrutschen des Haltemittels 4, wenn dieses im Abschnitt 15 zwischen den zwei ringförmigen Erhöhungen 14 festgelegt ist. Die ringförmigen Erhöhungen 14 selbst können ebenfalls das äußere Haltemittel 4 mit dem Adapter 5 verbinden, indem sie beispielsweise mit dem Haltemittel 4 formschlüssig zusammenwirken.

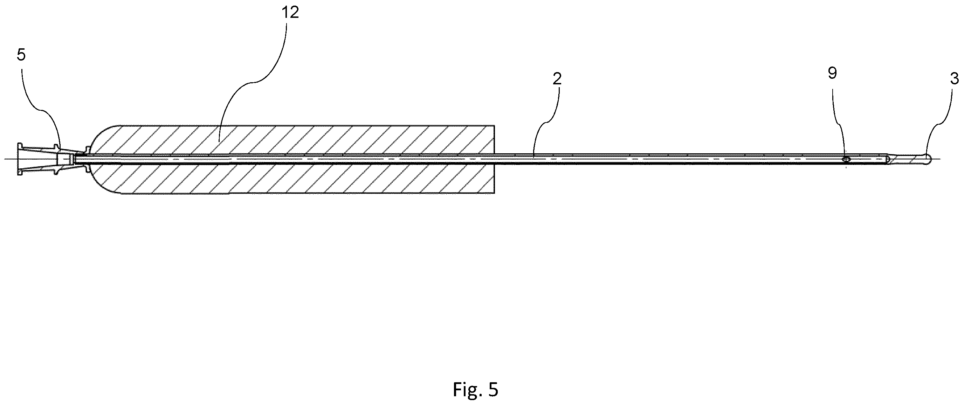

Fig. 5 zeigt eine Kathetervorrichtung 1 mit einem Katheterschlauch 2 und einem Adapter 5 gemäß Figur 4 im eingeführten Zustand. Der Katheterschlauch 2 ist in die Harnröhre des Penis 12 eingeführt. Ein äußeres Haltemittel 4 ist nicht gezeigt. Zur Fixierung des Katheterschlauchs 2 kann das äußere Haltemittel 4 an dem Abschnitt 15 des Adapters 5 festgelegt werden oder mit den ringförmigen Erhöhungen 14 formschlüssig zusammenwirken. Das äußere Haltemittel 4 kann dann an dem Penis 12 oder einem Penisnahen Körperteil, wie etwa einem Oberschenkel, befestigt werden, um den Katheterschlauch 2 zu fixieren. Die Stützstruktur 7 kann direkt an der Penisspitze anliegen oder der Penisspitze vorgelagert sein, ohne direkt an dieser anzuliegen.

Hinsichtlich weiterer vorteilhafter Ausgestaltungen der erfindungsgemäßen Kathetervorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Kathetervorrichtung lediglich zur Erörterung der beanspruchten Lehre dienen, diese jedoch nicht auf die Ausführungsbeispiele einschränken.

### Bezugszeichenliste

- 1: Kathetervorrichtung
- 2: Katheterschlauch
- 3: Einführhilfe
- 4: äußeres Haltemittel
- 5: Adapter
- 6: Aufnahme Katheterschlauch
- 7: Stützstruktur
- 8: Auslass
- 9: Ablaufauge(n)
- 10: Konnektor
- 11: Schlauchansatz
- 12: Penis
- 13: Durchflussöffnung
- 14: ringförmige Erhöhungen
- 15: Abschnitt zur Verbindung mit einem äußeren Haltemittel

## Patentansprüche

1. Kathetervorrichtung (1) zur Harnableitung mit einem flexiblen Katheterschlauch (2) und einem äußeren Haltemittel (4) zum Fixieren von mindestens einem Teil des Katheterschlauchs (2) in der Harnröhre und der Blase im eingeführten Zustand.

2. Kathetervorrichtung nach Anspruch 1, wobei das äußere Haltemittel (4) auf Grundlage von Adhäsion, Formschluss, Stoffschluss, Haftreibung, Vakuum, Klettwirkung oder Kombinationen davon wirkt.

3. Kathetervorrichtung nach Anspruch 1 oder 2, wobei das äußere Haltemittel (4) als eine elastische Hülle oder ein elastischer Schlauch ausgebildet ist, die/der über den Penis (12) ziehbar und/oder abrollbar ist.

4. Kathetervorrichtung nach Anspruch 1 oder 2, wobei das äußere Haltemittel (4) als Klebestreifen, Klettvorrichtung, Gummiband, Fixierbinde, Halteband oder Kombinationen davon ausgebildet ist.

5. Kathetervorrichtung nach einem der Ansprüche 1 bis 4, wobei der Katheterschlauch (2) einen Adapter (5) mit einer Aufnahme (6) für das nicht-einführseitige Ende des Katheterschlauchs (2) aufweist und wobei der Adapter (5) an diesem Ende fest angeordnet ist.

6. Kathetervorrichtung nach Anspruch 5, wobei der Adapter (5) auf der dem Penis (12) zugewandten Seite eine Stützstruktur (7) zur Abstützung von zumindest einem Teil des Penis (12) aufweist.

7. Kathetervorrichtung nach Anspruch 5 oder 6, wobei das äußere Haltemittel (4) zumindest teilweise mit dem Adapter (5) lösbar oder unlösbar verbunden ist.

8. Kathetervorrichtung nach einem der Ansprüche 5 bis 7, wobei der Adapter (5) auf der dem Penis (12) abgewandten Seite einen Auslass (8) zur Harnableitung und/oder Konnektierung mit einem Urinbeutel aufweist.

9. Kathetervorrichtung nach einem der Ansprüche 1 bis 8, wobei der Katheterschlauch (2) eine gelbeschichtete oder hydrophile Oberfläche aufweist.

10. Kathetervorrichtung nach einem der Ansprüche 1 bis 9, wobei der Katheterschlauch (2) eine am einführseitigen Ende des Katheterschlauchs fest angeordnete Einführhilfe (3) zum Einführen in die Harnröhre bzw. zum Durchführen in die Blase aufweist.

11. Kit, umfassend
- einen Katheter mit einem flexiblen Katheterschlauch (2), und
- ein äußeres Haltemittel (4) zum Fixieren von mindestens einem Teil des Katheterschlauchs (2) in der Harnröhre und der Blase im eingeführten Zustand.
